# EUROPÄISCHE PATENTSCHRIFT

(11) **EP 4 253 353 B1**
(45) Veröffentlichungstag und Bekanntmachung des Hinweises auf die Patenterteilung: **22.10.2025**
(21) Anmeldenummer: 22165010.4
(22) Anmeldetag: 29.03.2022
(51) Int. Cl.: C07C 7/04, C07C 9/12, C07C 9/10, C07C 9/08, C07C 7/163, C07C 7/12, B01D 53/02, B01D 53/04

(54) **VERFAHREN ZUR ENTFERNUNG FREMDGERUCHBILDENDER SUBSTANZEN AUS KOHLENWASSERSTOFFSTRÖMEN**
METHOD FOR REMOVING SUBSTANCES WHICH CREATE UNWANTED FRAGRANCES FROM HYDROCARBON FLOWS
PROCÉDÉ D'ÉLIMINATION DES SUBSTANCES ODORANTES ÉTRANGÈRES DE L'ÉCOULEMENT D'HYDROCARBURES

(43) Veröffentlichungstag der Anmeldung: 04.10.2023
(73) Patentinhaber: Evonik Oxeno GmbH & Co. KG, 45772 Marl (DE)
(72) Erfinder: PEITZ, Stephan, 45739 Oer-Erkenschwick (DE); STOCHNIOL, Guido, 45721 Haltern am See (DE); RIX, Armin Matthias, 45770 Marl (DE); BÖSE, Matthias, 46348 Raesfeld (DE); SIX, Tanita Valérie, 44309 Dortmund (DE); ALTHOFF, Martin, 45772 Marl (DE); BOLL, Ralf, 46286 Dorsten (DE); LOPEZ-FERNANDEZ, Helena, 44649 Herne (DE)
(74) Vertreter: Evonik Patent Association

(56) Entgegenhaltungen:
- EP-A1- 1 069 101
- US-A1- 2016 347 690

## Beschreibung

Gegenstand der Erfindung ist ein Verfahren zur Reinigung eines Kohlenwasserstoffstroms, der zumindest Mittelsieder wie Cx-Alkane, Cx-Olefine, Leichtsieder wie Cx-1-Kohlenwasserstoffe und Schwersieder wie Cx+1-Kohlenwasserstoffe, mit x = 3 oder 4, enthält. Das Verfahren umfasst eine Leichtsiederabtrennung und eine Schwersiederabtrennung, wobei die Behandlung der verbliebenen Mittelsieder in Anwesenheit von Wasserstoff durchgeführt wird und dadurch eine Hydrierung der vorhandenen Olefine sowie die weitestgehende Entfernung von weiteren unerwünschten Stoffen stattfindet.

Die Entfernung von unerwünschten und/oder geruchsbildenden Stoffen ist insbesondere für kosmetisch oder medizinisch verwendete Treibgase, also zumeist niedermolekulare Alkane wie Propan, Isobutan oder n-Propan, ein unerlässlicher Schritt. Der Begriff geruchsbildender Stoff ist in diesem Zusammenhang jeder Stoff oder Verbindung, die nicht dem Stoff entspricht, der das Treibgas darstellt. Jede Belastung mit reaktiven Komponenten wie Olefinen, aber auch jede geruchliche Belastung mit anderen Stoffen oder Verbindungen muss vermieden werden, da es sich zumeist um körpernahe Anwendungen handelt.

In der Literatur finden sich vielfältige Verfahren zur Aufreinigung von Treibgasen wie Propan, Isobutan oder n-Butan. Oftmals werden verschiedene Varianten der Sorption eingesetzt, beispielsweise die Adsorption über einem Adsorber wie Aktivkohle, bei der ein Gasstrom über den Adsorber geleitet wird und so bestimmte Stoffe durch den Adsorber adsorbiert und aus damit aus dem Gasstrom entfernt werden. Der Begriff Sorption umfasst im Rahmen der vorliegenden Erfindung sowohl die Physisorption als auch die Chemisorption, da diese beiden Sorptionsarten nicht immer klar voneinander getrennt werden können.

Die US 2016/347690 A1 offenbart beispielsweise ein Verfahren zur Reinigung von Kohlenwasserstoffströmen durch Kontaktieren mit einem festen Sorptionsmittel, um dadurch zumindest teilweise schwefelhaltige Verunreinigungen zu entfernen. Der Kohlenwasserstoffstrom liegt währenddessen ausschließlich im flüssigen Zustand vor. Als Sorptionsmittel wird eine Mischung aus Kupferoxid, Zinkoxid und Aluminiumoxid in einer bestimmten Zusammensetzung eingesetzt. Die Reinigung selbst wird in Gegenwart einer geringen Menge Wasserstoff durchgeführt.

Die EP 1 069 101 A1 offenbart weiterhin ein Verfahren zur Herstellung von C5-/C6-Olefinen aus einem olefinischen C4-Kohlenwasserstoffstrom durch Metathese. Vor der Metathesereaktion wird der dabei eingesetzte C4-Kohlenwasserstoffstrom durch Durchfahren eines Adsorber-Schutzbettes von Verunreinigungen befreit.

Bei den bekannten Sorptionsverfahren besteht allerdings das Problem, dass die eingesetzten Adsorber mit der Zeit wieder gereinigt werden muss, um die adsorbierten Stoffe wieder auszutreiben. Dies kann sowohl häufige Adsorberwechsel als auch hohe Regenerations- bzw. Beschaffungskosten für den Adsorber nach sich ziehen. Weiterhin muss meist eine zusätzliche Hydrierung der im Rohstoff enthaltenen Olefine vorgenommen werden, da diese nur unzureichend auf Adsorbern binden und nicht vollständig entfernt werden können. Zusätzlich müssen sowohl Leichtsieder in einer Leichtsiederabtrennung als auch Schwersieder in einer separaten Schwersiederabtrennung entfernt werden, um die gewünschte Kettenlängenverteilung im Treibgas zu erhalten. Das alles führt neben den hohen Kosten zu sehr aufwendigen und vielstufigen Verfahren mit komplexer Verschaltung von Anlagenteilen.

Die Aufgabe der vorliegenden Erfindung war deshalb die Bereitstellung eines Reinigungsverfahrens mit möglichst einfacher Verschaltung der jeweiligen Anlagenteile. Weiterhin sollte ein möglichst kostengünstiges Verfahren bereitgestellt werden.

Die Aufgabe konnte durch das hier beschriebene Verfahren gemäß Anspruch 1 gelöst werden. Bevorzugte Ausgestaltungen des Verfahrens sind in den Unteransprüchen angegeben. Das erfindungsgemäße Verfahren ist ein Verfahren zur Reinigung eines Kohlenwasserstoffstroms, welcher zumindest Mittelsieder wie Cₓ-Alkane, Cₓ-Olefine, Leichtsieder wie Cₓ₋₁-Kohlenwasserstoffe und Schwersieder wie Cₓ₊₁-Kohlenwasserstoffe, wobei x = 3 oder 4 ist, enthält, wobei das Verfahren die folgenden Schritte umfasst:
a) Abtrennung zumindest eines Teils der Leichtsieder und Abtrennung zumindest eines Teils der Schwersieder in einer einzigen Destillationskolonne unter Erhalt eines Mittelsieder-Intermediats;
b) Entfernen zumindest eines Teils der verbliebenen Leichtsieder und/oder zumindest eines Teils der verbliebenen Schwersieder aus dem aus Schritt a) erhaltenen Mittelsieder-Intermediat mittels Sorption in einer Sorptionseinheit unter Erhalt eines gereinigten Kohlenwasserstoffstroms, der zu mehr als 98 Gew.-% Cₓ-Alkane enthält, dadurch gekennzeichnet, dass
die Sorption in Schritt b) in Gegenwart von Wasserstoff durchgeführt wird, und dass in der Sorptionseinheit zusätzlich eine Hydrierung zumindest eines Teils der vorhandenen Olefine stattfindet.

Der zu reinigende Kohlenwasserstoffstrom ist ein Cₓ-Kohlenwasserstoffstrom mit x = 3 oder 4. Derartige Ströme enthalten sowohl Cₓ-Alkane und Cₓ-Olefine, aber auch Leichtsieder wie Cₓ₋₁-Kohlenwasserstoffe und Schwersieder wie Cₓ₊₁-Kohlenwasserstoffe. Solche Ströme sind großtechnisch verfügbar und fallen beispielsweise als Abfallprodukte in chemischen Prozessen an, können jedoch aufgrund der Anwesenheit der Leicht- und Schwersieder, sowie weiterer unerwünschter Stoffe nur schwer kommerziell genutzt oder verkauft werden. Um Ströme zu erhalten, die mehr als 98 Gew.-% Cₓ-Alkane zu erhalten, werden die Ströme erfindungsgemäß aufgereinigt.

Der zu reinigende Kohlenwasserstoffstrom ist demnach ein C3-Kohlenwasserstoffstrom oder ein C4-Kohlenwasserstoffstrom. Erfindungsgemäße C3-Kohlenwasserstoffströme enthalten zumindest Propen, Propan, C2-Kohlenwasserstoffe (z. B. Ethen, Ethan) und C4-Kohlenwasserstoffe (z. B. Butan, Buten). Ein derartiger C3-Kohlenwasserstoffstrom wird im Rahmen der vorliegenden Erfindung auch als (zu reinigender) Propanstrom bezeichnet. Erfindungsgemäße C4-Kohlenwasserstoffströme enthalten zumindest Buten (z. B. 1-Buten, 2-Buten, ggf. Isobuten), Butan, C3-Kohlenwasserstoffe (z. B. Propen, Propan) und C5-Kohlenwasserstoffe (z. B. Penten, Pentan). Ein derartiger C4-Kohlenwasserstoffstrom wird im Rahmen der vorliegenden Erfindung auch als (zu reinigender) Butanstrom oder Isobutanstrom bezeichnet. In einer bevorzugten Ausführungsform wird als zu reinigender Kohlenwasserstoffstrom ein Propanstrom eingesetzt.

Die Zusammensetzung des Propanstroms ist grundsätzlich nicht auf bestimmte Zusammensetzung limitiert. Voraussetzung ist lediglich, dass die einzelnen Komponenten gemäß Anspruch 1 vorhanden sind. Erfindungsgemäß kann der Propanstrom aber von 0,0001 bis zu 3 Gew.-% C2-Kohlenwasserstoffe, von 0,01 bis zu 20 Gew.-% Propen und von 0,0001 bis zu 20 Gew.-% C4-Kohlenwasserstoffe enthalten. Weiterhin kann der Propanstrom zusätzlich unter anderem geringe Mengen Methanthiol, Ethanthiol, Dimethylsulfid, Dimethyldisulfid und/oder Schwefelwasserstoff (jeweils bis zu 100 Gew-ppm) enthalten. Der Propanstrom kann weitere Stoffe oder Verbindungen in Spuren enthalten, die nicht einzeln aufgezählt werden können. Einige dieser Stoffe können einen wahrnehmbaren Geruch haben. Ihre Anwesenheit ist unerwünscht und wird durch das hier beschriebene Verfahren minimiert.

Im ersten Schritt a) des erfindungsgemäßen Verfahrens wird der zu reinigende Kohlenwasserstoffstrom, insbesondere der Propanstrom oder der Butanstrom, einer Destillationskolonne zugeführt, wo zumindest ein Teil der Leichtsieder und zumindest ein Teil der Schwersieder abgetrennt werden. Diese Abtrennung erfolgt in der gleichen Destillationskolonne. Es versteht sich, dass die vorhandenen Leichtsieder dabei am Kopf und die vorhandenen Schwersieder im Sumpf anfallen. Der durch die zumindest teilweise erfolgte Leichtsieder- und Schwersiederabtrennung vorgereinigte Kohlenwasserstoffstrom, hier Mittelsieder-Intermediat genannt, kann dann insbesondere als Seitenstrom bzw. als Mittelsiederfraktion abgenommen werden.

Die für die Abtrennung zumindest eines Teils der Leichtsieder und für die Abtrennung zumindest eines Teils der Schwersieder in Schritt a) eingesetzte Destillationskolonne kann grundsätzlich beliebig ausgestaltet sein, solange die Funktion, also die gleichzeitige Abtrennung von Leicht- und Schwersiedern gewährleistet wird. In einer bevorzugten Ausführungsform besteht die in Schritt a) eingesetzte Destillationskolonne aus einem oberen Abschnitt, einem mittleren Abschnitt und einem unteren Abschnitt.

Der obere Abschnitt umfasst dabei den Kopf der Destillationskolonne, an dem ein Strom anfällt, der die Leichtsieder enthält, und mindestens eine Trennstufe. Eine Trennstufe kann dabei eine oder mehrere Trennböden, Packungen oder Füllkörperbetten oder Kombinationen daraus enthalten. Der am Kopf des oberen Abschnitts der Destillationskolonne anfallende und abgenommene Strom, der die Leichtsieder enthält, wird vorzugsweise zu einem Kondensator geführt und dort zumindest teilweise kondensiert. Sowohl der nicht kondensierte Teil des Stroms als auch der kondensierte Teil des Stroms enthalten jeweils Leichtsieder. Der nicht kondensierte Teil wird dann zumindest teilweise aus dem Verfahren ausgeschleust, und kann z. B. ins Abgas chemischer Produktionsanlagen geführt werden. Der kondensierte Teil kann noch signifikante Mengen an Cₓ-Alkanen, Cx-Olefinen oder Schwersiedern enthalten und wird deshalb vorzugsweise zumindest zum Teil zur Trennwandkolonne zurückgeführt. Dabei ist es bevorzugt, wenn der zurückgeführte kondensierte Teil des Stroms auf oder in die erste Trennstufe des oberen Abschnitts der Destillationskolonne geführt wird.

Der mittlere Abschnitt der Destillationskolonne umfasst mindestens eine Trennstufe. Eine Trennstufe kann dabei eine oder mehrere Trennböden, Packungen oder Füllkörperbetten oder Kombinationen daraus enthalten. In einer bevorzugten Ausführungsform der vorliegenden Erfindung ist die in Schritt a) eingesetzte Destillationskolonne eine Trennwandkolonne, d. h. die Destillationskolonne weist eine Trennwand auf, die sich vertikal zumindest über einen Teil des mittleren Abschnitts der Destillationskolonne erstreckt. Vorzugsweise erstreckt sich die Trennwand vertikal über die gesamte Höhe des mittleren Abschnitts. Durch die Trennwand wird der mittlere Abschnitt zumindest teilweise in zwei voneinander getrennte Bereiche geteilt, wovon einer als Zulaufteil und der andere als Ablaufteil bezeichnet wird. Die Trennwand verläuft also radial von Wandung zu Wandung innerhalb der Kolonne, um die beiden voneinander getrennte Bereiche zu schaffen. Der Zulaufteil definiert sich darüber, dass sich an diesem Teil des mittleren Abschnitts der Zulauf zur Destillationskolonne befindet, durch den die Destillationskolonne mit dem zu reinigenden Kohlenwasserstoffstrom gespeist wird. Der Ablaufteil definiert sich darüber, dass an diesem des mittleren Abschnitts der Ablauf der Destillationskolonne befindet, durch den das Mittelsieder-Intermediat aus der Destillationskolonne entnommen wird. Sowohl im Zulaufteil als auch im Ablaufteil liegt mindestens eine Trennstufe vor. Es können jedoch auch jeweils mehr als eine Trennstufe im Zulaufteil und im Ablaufteil vorhanden sein. Dabei kann die Anzahl der Trennstufen im Zulaufteil und im Ablaufteil gleich oder unterschiedlich voneinander sein.

Der mittlere Abschnitt der Destillationskolonne weist am Ablaufteil vorzugsweise einen Seitenabzug, an dem das Intermediat aus Schritt a) abgenommen wird und von dort zur Sorption in Schritt b) geleitet wird. Weiterhin bevorzugt enthält der mittlere Abschnitt der Destillationskolonne einen Zulauf für den zu reinigenden Kohlenwasserstoffstrom, welcher am Zulaufteil angeordnet ist. Vor Eintritt in die Destillationskolonne kann der zu reinigende Kohlenwasserstoffstrom einen Wärmetauscher durchlaufen. Dadurch wird der zu reinigende Strom vorgewärmt wird, bevor er der Trennwandkolonne zugeführt wird. Das Aufwärmen im Wärmetauscher erfolgt vorzugsweise durch Wärmeaustausch mit dem aus der Destillationskolonne entnommenen Intermediat. Das hat den Vorteil, dass das Mittelsieder-Intermediat abgekühlt wird, bevor es in die Sorption in Schritt b) gelangt und spart außerdem Energie für das Aufheizen des zu reinigenden Kohlenwasserstoffstroms.

Der untere Abschnitt umfasst den Sumpf der Destillationskolonne, an dem ein Strom anfällt, der die Schwersieder enthält, und mindestens eine Trennstufe. Eine Trennstufe kann dabei eine oder mehrere Trennböden, Packungen oder Füllkörperbetten oder Kombinationen daraus enthalten. Der aus dem mittleren Teil der Destillationskolonne nach unten gelangte Strom wird dabei zunächst die mindestens eine Trennstufe passieren und von dort in den Sumpf gelangen. Unter der letzten Trennstufe des unteren Abschnitts der Destillationskolonne wird ein Strom abgenommen, durch einen Sumpfheizer gefahren, wodurch zumindest ein Teil des Stroms verdampft, und dann zumindest teilweise in den Sumpf zurückgeführt. Der Sumpfheizer dient in der erfindungsgemäßen Destillationskolonne insbesondere dem Eintragen zumindest eines Teils der zur Auftrennung notwendigen Energie. In einer bevorzugten Ausführungsform der vorliegenden Erfindung wird der Strom nach Durchfahren des Sumpfheizers aufgetrennt, wovon ein Teil in den Sumpf der Destillationskolonne zurückgeführt und der andere Teil als Schwersieder aus dem Verfahren ausgeschleust wird. Es ist aber auch möglich, dass der Schwersieder direkt aus dem Sumpf der Destillationskolonne, d. h. ohne vorherige Trennung entnommen und aus dem Verfahren ausgeschleust wird. Er könnte dann beispielsweise einer thermischen Verwertung zugeführt werden.

Das aus der Abtrennung der Leichtsieder und der Schwersieder in Schritt a) erhaltene Mittelsieder-Intermediat wird anschließend in Schritt b) zu einer Sorptionseinheit geführt, um restliche Leichtsieder und restliche Schwersieder zumindest teilweise aus dem Strom zu entfernen und zumindest einen Teil der vorhandenen (leichtsiedenden, mittelsiedenden oder schwersiedenden) Olefine zu hydrieren. Aus diesem Schritt b) wird dann ein gereinigter Kohlenwasserstoffstrom erhalten, der zu mehr als 98 Gew.-%, bevorzugt mehr als 99 Gew.-%, besonders bevorzugt mehr als 99,5 Gew.-% Cₓ-Alkane enthält. In einer bevorzugten Ausführungsform der vorliegenden Erfindung enthält der aus dem Schritt b) erhaltene gereinigten Kohlenwasserstoffstrom maximal 2 Gew.-%, bevorzugt maximal 1 Gew.-%, besonders bevorzugt maximal 0,5 Gew.-% an Cx+1-Kohlenwasserstoffen. Weiterhin bevorzugt enthält der aus dem Schritt b) erhaltene gereinigten Kohlenwasserstoffstrom maximal 200 Gew.-ppm an Cx-Olefin. Wird bei dem Verfahren ein C3-Kohlenwasserstoffstrom gereinigt, wird vorzugsweise ein Propanstrom erhalten, der maximal 2 Gew.-%, bevorzugt maximal 1 Gew.-%, besonders bevorzugt maximal 0,5 Gew.-% an C4-Kohlenwasserstoffen, insbesondere iso-Butan und n-Butan enthält. Der Propanstrom enthält dann weiterhin bevorzugt maximal 200 Gew.-ppm Propen.

Um einen ausreichenden Teil der Olefine im Schritt b) zu hydrieren wird eine angemessene Menge an Wasserstoff zugegeben. Die exakte Menge kann je nach gewünschter Umsetzung bestimmt werden. Das ist dem Fachmann geläufig. Der Wasserstoff kann dabei zum Mittelsieder-Intermediat im Zulauf zur Sorptionseinheit dosiert werden. Um dabei eine ausreichende Löslichkeit des Wasserstoffs zu gewährleisten, sollte der Olefingehalt im Mittelsieder-Intermediat 5 Gew.-% nicht überschreiten. Dies kann entweder durch geeignete Rohstoffauswahl des zu reinigenden Kohlenwasserstoffstroms oder durch eine entsprechende Abtrennung der Olefine im vorgeschalteten Schritt a) erfolgen.

Die Sorption in Schritt b) wird insbesondere mit einem geeigneten Sorptionsmittel durchgeführt. Das erfindungsgemäß bevorzugt eingesetzte Sorptionsmittel umfasst ein poröses Trägermaterial, insbesondere SiO₂, welches auf seiner Oberfläche mit Nickel und Zinkoxid belegt ist. Graphit kann bei der Formgebung als Gleitmittel eingesetzt werden und kann daher ebenfalls enthalten sein. Sonstige Komponenten, beispielsweise Spuren von Titandioxid oder Aluminiumoxid können aus produktionstechnischen Gründen enthalten sein.

Das Sorptionsmittel kann demnach die folgende, sich zu 100 Gew.-% ergänzende Zusammensetzung aufweisen:
▪ Nickel: 15 bis 65 Gew.-%;
▪ Zinkoxid: 5 bis 40 Gew.-%;
▪ Siliciumdioxid: 5 bis 75 Gew.-%;
▪ Graphit: 0 bis 5 Gew.-%;
▪ sonstige Komponenten 0 bis 1 Gew.-%.

Als Trägermaterial wird vorzugsweise Fällungskieselsäure verwendet. Aufgrund der feinen Partikelverteilung und der hohen spezifischen Oberfläche ergeben sich an elementarem Nickel pyrophore Eigenschaften, d. h. das Sorptionsmittel kann sich bei 20°C und Luftatmosphäre selbst entzünden. Dies erschwert zwar die Handhabung des Sorptionsmittels, steigert aber seine Effektivität. Deswegen ist die Verwendung von einem auf Ni/ZnO/SiO₂-basierenden System mit pyrophoren Eigenschaften als Sorptionsmittel bevorzugt.

Die pyrophoren Eigenschaften ergeben sich insbesondere dann, wenn man eine metallische Nickeloberfläche von mindestens 3 m²/g bezogen auf den Nickelgehalt des Sorptionsmittels vorsieht. Dies begünstigt die Adsorptionswirkung und bedingt zugleich die pyrophoren Eigenschaften. Die Oberfläche wird mittels Wasserstoff-Chemiesorption gemessen. Bevorzugt wird also ein Sorptionsmittel eingesetzt, das eine metallische Nickeloberfläche von größer als 3 m²/g, bevorzugt von größer als 5 m²/g, besonders bevorzugt von größer als 7 m²/g aufweist, jeweils bezogen auf das Gesamtgewicht an Nickel in dem Sorptionsmittel.

Um das Ni/ZnO/SiO2-System, das als Sorptionsmittel eingesetzt wird, zu aktivieren, ist es vor Gebrauch mit einem Wasserstoffstrom bei einer Temperatur von 150°C bis 400°C, bevorzugt 180°C bis 280°C, insbesondere 200°C bis 240°C zu spülen bzw. zu reduzieren. Die Aktivierung mit Wasserstoff kann in-situ oder ex-situ erfolgen, also am späteren Einsatzort oder davon entfernt, nach der Herstellung des Sorptionsmittels. Sofern das Sorptionsmittel desaktiviert, kann es mit einer weiteren Wasserstoffspülung wieder reaktiviert werden.

Ein Beispiel für ein geeignetes Sorptionsmittel ist der von der Evonik Industries AG erhältliche Katalysator Octolyst^{®} H10126. Ein besonderer Vorteil des Verfahrens liegt mithin darin, dass das für die Sorption in Schritt b) eingesetzte Sorptionsmittel als Katalysator kommerziell verfügbar ist und deswegen nicht erst hergestellt werden muss. Das Sorptionsmittel wird dabei üblicherweise in oxidierter Form angeliefert, die eine Handhabung bei Raumtemperatur an der Luft zulässt. Vor dem Einsatz muss das Sorptionsmittel daher wie erwähnt durch eine Nachreduktion mit Wasserstoff aktiviert werden. Nach dem Einsatz muss das Sorptionsmittel durch Oxidation mit Luft stabilisiert werden, so dass es in einfacher Weise entnommen werden kann.

Sofern das Sorptionsmittel nicht zugekauft werden soll, kann seine Herstellung grundsätzlich durch die folgenden Schritte geschehen:
1. Bereitstellen eines porösen Gerüstmaterials aus Siliciumdioxid;
2. Vermengen des Gerüstmaterials mit Nickelcarbonat und Zinkoxid;
3. Thermische Zersetzung des Nickelcarbonats zu NiO;
4. Reduktion unter Wasserstoffzufuhr zu metallischem Nickel.

Die Schritte 3. und 4. können zur Vermeidung von Exothermiespitzen auch in einem Schritt erfolgen, da 3. endotherm und 4. stark exotherm sind. In einer bevorzugten Ausführungsform wird also in einem Schritt das eingesetzte Nickelcarbonat (NiCO₃) thermisch zersetzt und parallel entstehendes NiO mit Wasserstoff zu metallischem Nickel reduziert. In einer zweiten Ausführungsform können beide Schritte separat erfolgen. In beiden Fällen kann zur Aktivierung auch ein Gemisch aus Stickstoff und Wasserstoff verwendet werden, dessen Wasserstoffgehalt im Laufe der Aktivierung gesteigert wird.

Das Sorptionsmittel wird vorzugsweise in einem Reaktor als Bett aufgeschüttet und von dem zu reinigenden Kohlenwasserstoffgemisch durchströmt. Entsprechende anlagentechnische Anordnungen sind dem Fachmann bekannt. Die Einhaltung einer bestimmten Temperatur während der Sorption in Schritt b) kann Einfluss für das Reinigungsvermögen des Sorptionsmittel haben. Es ist vorteilhaft, wenn die Temperatur bei der Sorption in Schritt b) des erfindungsgemäßen Verfahrens zwischen 10°C und 150°C, bevorzugt zwischen 20°C und 130°C und besonders bevorzugt zwischen 30°C und 120°C beträgt.

Um eine besonders effektive Reinigung zu erreichen und Betriebsunterbrechungen durch Wechsel des Sorptionsmittels zu vermeiden, empfiehlt es sich, mehrere Reaktoren einzusetzen, die derart revolvierend geschaltet werden können, dass immer ein Reaktor mit ausreichend frischem Sorptionsmittel für den Schritt b) genutzt werden kann. Mindestens ein Behälter kann dabei, ohne den zu reinigenden Strom zu unterbrechen, herausgenommen und das in ihm befindliche Material gespült und entnommen werden, danach erfolgt in analoger Weise die Neubefüllung.

Wichtig ist, dass sich das aus Schritt a) erhaltene Intermediat während des Kontakts mit dem Sorptionsmittel ausschließlich im flüssigen Zustand befindet. Im angegebenen Temperaturbereich wird dies insbesondere durch einen Druck zwischen 5 bis 35 bar gewährleistet. Letztendlich kommt es auf den Druck jedoch nicht an, solange sich das Intermediat im flüssigen Zustand befinden. Die Raum-/Zeit-Belastung (weight hour space velocity - WHSV) wird dann vorzugsweise zwischen 0.5 und 15 h⁻¹ gewählt. Die Betten bestehen aus einer Schüttung des jeweiligen Sorptionsmittels mit einer Schüttdichte im Bereich von 0.7 bis 1.5 kg/m³, bevorzugt etwa 1.15 kg/m³.

Bei den Verunreinigungen, die erfindungsgemäß aus dem aus Schritt a) erhaltenen Intermediat entfernt werden sollen, handelt es sich vorzugsweise um organische Schwefelverbindungen, die in der nachfolgenden Aufarbeitung des Kohlenwasserstoffgemisches als Katalysatorgift wirken. Zu katalysatorschädlichen, organischen Schwefelverbindungen, die in den üblicherweise erhältlichen Rohstoffströmen enthalten sind, gehören insbesondere Thiole mit der allgemeinen Formel R-SH, Disulfide mit der allgemeinen Formel R-S-S-R', Sulfide mit der allgemeinen Formel R-S-R' und substituierte oder unsubstituierte schwefelhaltige Heterozyklen, wie insbesondere Thiophene und/oder Thiolane. In den oben angegebenen Strukturformeln können R und R' gleiche oder unterschiedliche Alkyl-, Aryl-, Cycloalkyl-, oder Alkenyl-Reste sein, wobei es sich bei R und R' insbesondere um Methyl-, Ethyl-, Propyl-, Butyl-, Phenyl-, Cyclohexyl- oder Butenyl-Reste handelt.

Die Sorption der der vorgenannten Verunreinigungen ist irreversibel. Aus diesem Grund kann das erfindungsgemäß eingesetzte Sorptionsmittel nicht regeneriert werden. Dies bedeutet, dass stark verunreinigte Ströme die Sorptionsmittel rasch erschöpfen, sodass sie ausgetauscht werden müssen. Im Interesse des wirtschaftlichen Betriebs des Reinigungsverfahrens sollte der Gewichtsanteil der Verunreinigungen in dem verunreinigten Kohlenwasserstoffgemisch bezogen auf dessen Gesamtgewicht vorzugsweise kleiner als 0.2 Gew.-% sein. Besonders bevorzugt enthält das verunreinigte Kohlenwasserstoffgemisch weniger als 100 Gew.-ppm und besonders bevorzugt weniger als 10 Gew.-ppm Verunreinigungen, jeweils gerechnet als Schwefelatom. Bei einem solch geringen Verunreinigungsgrad lässt sich das Sorptionsmittel sehr lange betreiben und ermöglicht zudem eine nahezu restlose Entfernung der Katalysatorgifte. Die erfindungsgemäß vorgeschaltete Leicht- und Schwersiederabtrennung bewirkt einen solch geringen Verunreinigungsgrad, da bestimmte leicht- und schwersiedende Schwefelverbindungen bereits dort aus dem Cx-Strom abgetrennt werden und am Soprtionsbett nur noch sehr geringe Gehalte an Schwefelverbindungen ankommen.

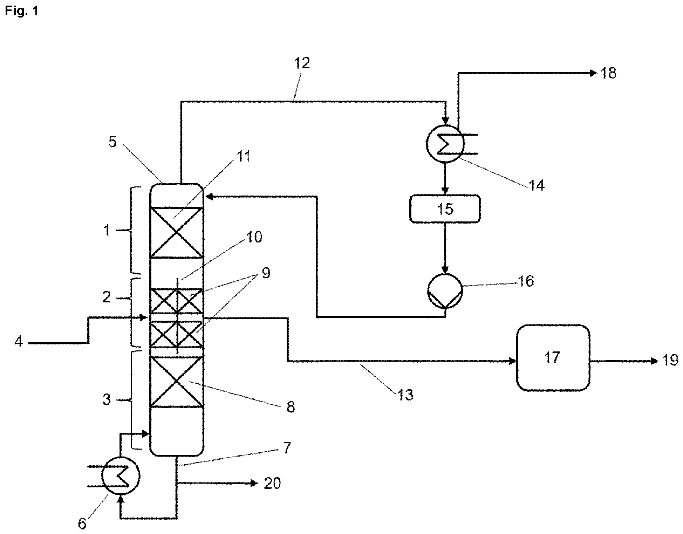

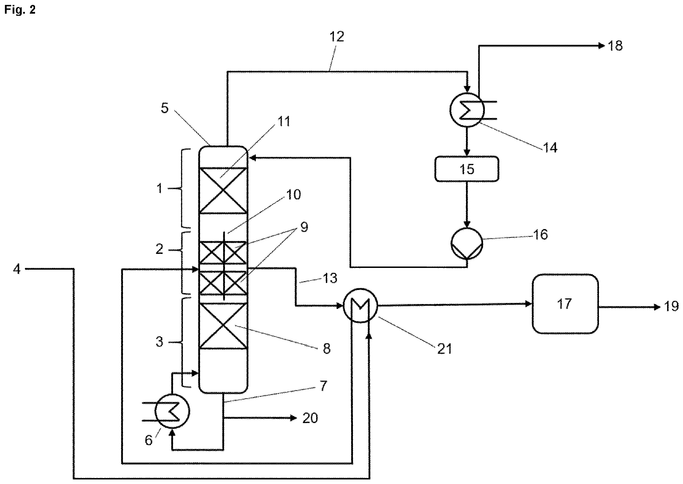

Bevorzugte exemplarische Ausführungsformen sind in den beiden Abbildungen Fig. 1 und Fig. 2 gezeigt. Es versteht sich, dass das erfindungsgemäße Konzept auch mit anderen Ausführungsformen realisieren lässt.

Fig. 1 zeigt eine Ausführungsform der vorliegenden Erfindung mit einer Destillationskolonne (5) für die Abtrennung in Schritt a) und einer Sorptionseinheit (17) für die Sorption in Schritt b). Die Destillationskolonne (5) weist dabei einen oberen Abschnitt (1), der eine Trennstufe (11) umfasst, einen mittleren Abschnitt (2) mit mehreren Trennstufen (9) und einer Trennwand (10) und einen unteren Abschnitt (3) auf, der ebenfalls eine Trennstufe (8) umfasst. In den mittleren Abschnitt (2) wird der zu reinigende Kohlenwasserstoffstrom (4) zugeführt gemäß dem erfindungsgemäßen Verfahren aufgetrennt. Im Sumpf wird eine Sumpffraktion (7) abgenommen, wovon ein Teil als Schwersieder (20) aus dem Verfahren entnommen werden. Die restliche Sumpffraktion wird dann über einen Sumpfheizer (6) erwärmt und zurück in die Destillationskolonne (5) geführt. Dadurch wird zumindest ein Teil der zur Trennung notwendigen Energie in das System eingetragen. Am Kopf der Destillationskolonne (5) wird eine Kopffraktion (12) abgenommen und zu einem Kondensator (14) geführt. Die nicht kondensierten Anteile werden als Leichtsieder (18) aus dem Verfahren ausgeschleust. Der kondensierte Teil wird über einen Behälter (15) und eine Rücklaufpumpe (16) zum oberen Abschnitt (1) der Destillationskolonne (5) zurückgeführt. Das bei der Abtrennung in Schritt a) anfallende Intermediat (13) wird als Seitenstrom angenommen und zur Sorption in der Sorptionseinheit (17) geführt. Aus der Sorptionseinheit wird dann der gereinigte Kohlenwasserstoffstrom (19) erhalten.

Fig. 2 zeigt eine weitere Ausführungsform der vorliegenden Erfindung. Der Ablauf und die anlagentechnische Gegebenheiten sind weitestgehend mit der Fig. 1 identisch. Der einzige Unterschied besteht darin, dass der zu reinigende Kohlenwasserstoffstrom (4) durch einen Wärmetauscher (21) geführt wird bevor der in die Destillationskolonne (5) gelangt. Dort findet eine Energieaustausch zwischen dem zu reinigenden Kohlenwasserstoffstrom (4) und dem Intermediat (13) statt, wodurch der zu reinigende Kohlenwasserstoffstrom (4) aufgewärmt wird. Insofern ist der Wärmetauscher (21) auch als Vorwärmung des zu reinigenden Kohlenwasserstoffstroms (4) zu verstehen.

### Beispiele

### Beispiel 1 (erfindungsgemäß)

In eine erfindungsgemäße Trennwandkolonne wird ein C3-Strom gefahren, der 90 Gew.-% Propan, 1,5 Gew.-% Propen, 1 Gew.-% Ethan, 7,5 Gew.-% Isobutan, sowie 2 mg/kg Schwefel in Form von Methanthiol enthält. Im Seitenabzug der Kolonne wird dabei ein vorgereinigter C3-Strom erhalten, der ca. 99 Gew.-% Propan und ca. 1 Gew.-% Propen, sowie 1,2 mg/kg Schwefel in Form von Methanthiol enthält. Das Massenstromverhältnis von Kolonnenfeed zu Seitenabzug beträgt ca. 1:0,8.

In ein nachgeschaltetes Reaktorfestbett wird ein Teil dieses Seitenabzuges gefahren. Als Sorptionsmittel wird der handelsübliche Katalysator Octolyst ^{®} H 10126 der Evonik Industries AG, enthaltend ca. 45 Gew.-% Ni, ca. 28 Gew-% ZnO, ca. 25 Gew.-% SiO₂ und ca. 2 Gew.-% Graphit, in Form von zylindrischen Kalotten-Tabletten 5 x 5 mm, mit einer mittels Wasserstoff-Chemisorption gemessenen Nickeloberfläche von 9 m²/g in ein Reaktionsrohr mit 1 cm Durchmesser und 23 g Inhalt gefüllt. Die Schüttdichte beträgt ca. 1.15 kg/dm³. Das Adsorptionsmittel wurde zuvor im Stickstoff-Wasserstoffstrom bei ca. 220°C aktiviert, wobei je nach Wärmetönung zunächst 1 Vol.- % Wasserstoff, am Ende 50 Vol.-% Wasserstoff zugemischt werden. Danach wurde das Katalysatorbett im Stickstoffstrom abgekühlt.

Das Bett wird durch Beheizung der Rohrwände auf eine Temperatur von 30° C gebracht und bei einem Druck von 24 bar mit dem Gemisch aus dem Seitenabzug der Trennwandkolonne durchströmt. Die Belastung der Adsorberbetten beträgt 370 g/h, der Schwefeleintrag also etwa 0.44 mg/h. Es wird so viel Wasserstoff zugeführt, dass dieser unter den gegebenen Bedingungen noch löslich ist.

Der Schwefel wird ausweislich der Analysen zunächst nahezu quantitativ aus dem Gemisch entfernt (Tabelle 1). Der Durchbruch von Schwefel erfolgt nach etwa 600 Stunden. Das Reinigungsbett hat zu diesem Zeitpunkt insgesamt ca. 0. 27 g Schwefel aufgenommen, entsprechend einer Aufnahme von 1.2 Gew.-% bezogen auf das frisch eingefüllte Sorptionsmittel. Bis zum Zeitpunkt des Schwefel-Durchbruchs wurde Propen vollständig hydriert.

Nach dem Ende dieses Versuchs wird das Bett mit warmem Stickstoff gespült und anschließend mit kaltem Stickstoff-Luft-Gemisch vorsichtig oxidiert, bis in reiner Luft keine wesentliche Wärmetönung mehr erfolgt. Das Sorptionsmittel kann im Wesentlichen unversehrt und mit noch hinreichender Festigkeit entnommen werden. Die Ergebnisse des Versuchs sind in Tabelle 1 niedergelegt.

**Tabelle 1: Ergebnisse von Beispiel 1**

| Mittlerer S-Gehalt [Gew.-%] Zulauf | Mittlerer S-Gehalt [Gew.-%] Austrag bis 600 h | Mittlere Abnahme S [Gew.-%] in Austrag gegenüber Zulauf bis 600 h |
|---|---|---|
| 0.00012 | 0.000003 | 97,5 |

## Patentansprüche

1. Verfahren zur Reinigung eines Kohlenwasserstoffstroms, welcher zumindest Cₓ-Alkane, Cₓ-Olefine, Leichtsieder wie Cₓ₋₁-Kohlenwasserstoffe und Schwersieder wie Cₓ₊₁-Kohlenwasserstoffe, wobei x = 3 oder 4 ist, enthält, wobei das Verfahren die folgenden Schritte umfasst:
a) Abtrennung zumindest eines Teils der Leichtsieder und Abtrennung zumindest eines Teils der Schwersieder in einer einzigen Destillationskolonne unter Erhalt eines Intermediats;
b) Entfernen zumindest eines Teils der verbliebenen Leichtsieder und/oder zumindest eines Teils der verbliebenen Schwersieder aus dem aus Schritt a) erhaltenen Intermediat mittels Sorption in einer Sorptionseinheit unter Erhalt eines gereinigten Kohlenwasserstoffstroms, der zu mehr als 98 Gew.-% Cₓ-Alkane enthält,
**dadurch gekennzeichnet, dass** die Sorption in Schritt b) in Gegenwart von Wasserstoff durchgeführt wird, und dass in der Sorptionseinheit zusätzlich eine Hydrierung zumindest eines Teils der vorhandenen Olefine stattfindet.

2. Verfahren nach Anspruch 1, wobei als zu reinigender Kohlenwasserstoffstrom ein C3-Kohlenwasserstoffstrom eingesetzt wird.

3. Verfahren nach Anspruch 1 oder 2, wobei die Destillationskolonne aus einem oberen Abschnitt, der den Kopf der Destillationskolonne und mindestens eine Trennstufe umfasst, einen mittleren Abschnitt, der mindestens eine Trennstufe umfasst, und einen unteren Abschnitt, der den Sumpf der Destillationskolonne und mindestens eine Trennstufe umfasst, aufweist.

4. Verfahren nach Anspruch 3, wobei die Destillationskolonne eine Trennwandkolonne ist, die im mittleren Abschnitt eine Trennwand aufweist, wodurch der mittlere Abschnitt in zwei voneinander getrennte Teile, den Zulaufteil und den Ablaufteil, aufgeteilt wird.

5. Verfahren nach Anspruch 4, wobei das aus der Abtrennung in Schritt a) erhaltene Intermediat über einen Seitenabzug am Ablaufteil der Destillationskolonne abgenommen wird.

6. Verfahren nach Anspruch 4 oder 5, wobei der zu reinigende Kohlenwasserstoffstrom, aus dem die Leichtsieder und die Schwersieder zumindest teilweise entfernt werden, am Zulaufteil der Trennwandkolonne zugeführt wird.

7. Verfahren nach einem der vorhergehenden Ansprüche, wobei ein Strom, der die Leichtsieder enthält, in der Trennwandkolonne über den Kopf der Kolonne und ein Strom, der die Schwersieder enthält, über den Sumpf der Kolonne abgenommen werden.

8. Verfahren nach Anspruch 7, wobei der am Kopf abgenommene Strom, der die Leichtsieder enthält, zu einem Kondensator geführt und dort zumindest teilweise kondensiert wird.

9. Verfahren nach Anspruch 8, wobei der nicht kondensierte Teil des Stromes aus dem Verfahren ausgeschleust wird.

10. Verfahren nach Anspruch 8 oder 9, wobei der kondensierte Teil des Stroms zur Trennwandkolonne zurückgeführt wird.

11. Verfahren nach Anspruch 10, wobei der zurückgeführte kondensierte Teil des Stroms auf die erste Trennstufe des oberen Abschnitts der Destillationskolonne gegeben wird.

12. Verfahren nach einem der vorhergehenden Ansprüche, wobei der zu reinigende Kohlenwasserstoffstrom einen Wärmetauscher durchläuft und vorgewärmt wird, bevor der Strom der Trennwandkolonne zugeführt wird.

13. Verfahren nach Anspruch 12, wobei das Aufwärmen im Wärmetauscher durch Wärmeaustausch mit dem Intermediat erfolgt.

14. Verfahren nach einem der vorhergehenden Ansprüche, wobei bei der Abtrennung in Schritt b) ein Sorptionsmittel eingesetzt wird, das ein poröses Trägermaterial umfasst, insbesondere SiO₂, welches auf seiner Oberfläche mit Nickel und Zinnoxid belegt ist.

15. Verfahren nach Anspruch 14, wobei das Sorptionsmittel die folgende, sich zu 100 Gew.-% ergänzende Zusammensetzung aufweist:
▪ Nickel: 15 bis 65 Gew.-%;
▪ Zinkoxid: 5 bis 40 Gew.-%;
▪ Siliciumdioxid: 5 bis 75 Gew.-%;
▪ Graphit: 0 bis 5 Gew.-%;
▪ sonstige Komponenten 0 bis 1 Gew.-%.

## Claims

1. Process for purifying a hydrocarbon stream comprising at least Cₓ alkanes, Cₓ olefins, low boilers such as Cₓ₋₁ hydrocarbons and high boilers such as Cₓ₊₁ hydrocarbons, where x = 3 or 4, wherein the process comprises the following steps:
a) separating off at least a portion of the low boilers and separating off at least a portion of the high boilers in a single distillation column to obtain an intermediate;
b) removing at least a portion of the remaining low boilers and/or at least a portion of the remaining high boilers from the intermediate obtained from step a) by means of sorption in a sorption unit to obtain a purified hydrocarbon stream containing more than 98% by weight of Cₓ alkanes,
**characterized in that** the sorption in step b) is conducted in the presence of hydrogen, and **in that** a hydrogenation of at least a portion of the olefins present additionally takes place in the sorption unit.

2. Process according to Claim 1, wherein the hydrocarbon stream used for purification is a C3 hydrocarbon stream.

3. Process according to Claim 1 or 2, wherein the distillation column consists of an upper section comprising the top of the distillation column and at least one separation stage, a middle section comprising at least one separation stage, and a lower section comprising the bottom of the distillation column and at least one separation stage.

4. Process according to Claim 3, wherein the distillation column is a dividing wall column having a dividing wall in the middle section, which divides the middle section into two separate parts: the feed section and the outlet section.

5. Process according to Claim 4, wherein the intermediate obtained from the separating-off in step a) is removed via a side draw in the outlet section of the distillation column.

6. Process according to Claim 4 or 5, wherein the hydrocarbon stream to be purified, from which the low boilers and the high boilers are at least partly removed, is fed in in the feed section of the dividing wall column.

7. Process according to any of the preceding claims, wherein a stream containing the low boilers is removed via the top of the column in the dividing wall column, and a stream containing the low boilers is removed via the bottom of the column.

8. Process according to Claim 7, wherein the stream removed at the top that contains the low boilers is guided to a condenser, where it is at least partly condensed.

9. Process according to Claim 8, wherein the uncondensed portion of the stream is discharged from the process.

10. Process according to Claim 8 or 9, wherein the condensed portion of the stream is recycled to the dividing wall column.

11. Process according to Claim 10, wherein the recycled condensed portion of the stream is introduced into the first separation stage of the upper section of the distillation column.

12. Process according to any of the preceding claims, wherein the hydrocarbon stream to be purified passes through a heat exchanger and is preheated before the stream is fed to the dividing wall column.

13. Process according to Claim 12, wherein the heating in the heat exchanger is effected by heat exchange with the intermediate.

14. Process according to any of the preceding claims, wherein a sorbent comprising a porous carrier material, especially SiO₂, coated with nickel and tin oxide on its surface is used in the separating-off in step b).

15. Process according to Claim 14, wherein the sorbent has the following composition that adds up to 100% by weight:
▪ nickel: 15% to 65% by weight;
▪ zinc oxide: 5% to 40% by weight;
▪ silicon dioxide: 5% to 75% by weight;
▪ graphite: 0% to 5% by weight;
▪ other components 0% to 1% by weight.

## Revendications

1. Procédé de purification d'un flux hydrocarboné, qui contient au moins des alcanes en Cₓ, des oléfines en Cₓ, des constituants à bas point d'ébullition, tels que des hydrocarbures en Cₓ₋₁, et des constituants à point d'ébullition élevé, tels que des hydrocarbures en Cₓ₊₁, x = 3 ou 4, le procédé comprenant les étapes suivantes :
a) séparation d'au moins une partie des constituants à bas point d'ébullition et séparation d'au moins une partie des constituants à point d'ébullition élevé dans une seule colonne de distillation avec obtention d'un intermédiaire ;
b) élimination d'au moins une partie des constituants à bas point d'ébullition résiduels et/ou d'au moins une partie des constituants à point d'ébullition élevé résiduels de l'intermédiaire obtenu de l'étape a) par sorption dans une unité de sorption avec obtention d'un flux hydrocarboné purifié qui contient des alcanes en Cₓ à raison de plus de 98% en poids,
**caractérisé en ce que** la sorption dans l'étape b) est réalisée en présence d'hydrogène et **en ce qu'**une hydrogénation d'au moins une partie des oléfines présentes a lieu en plus dans l'unité de sorption.

2. Procédé selon la revendication 1, un flux hydrocarboné en C3 étant utilisé comme flux hydrocarboné à purifier.

3. Procédé selon la revendication 1 ou 2, la colonne de distillation comportant une section supérieure, qui comprend la tête de la colonne de distillation et au moins un plateau de séparation, une section centrale, qui comprend au moins un plateau de séparation, et une section inférieure, qui comprend le fond de la colonne de distillation et au moins un plateau de séparation.

4. Procédé selon la revendication 3, la colonne de distillation étant une colonne à paroi de séparation qui présente, dans la section centrale, une paroi de séparation qui divise la section centrale en deux parties séparées l'une de l'autre, la partie d'admission et la partie d'évacuation.

5. Procédé selon la revendication 4, dans lequel l'intermédiaire obtenu à partir de la séparation dans l'étape a) est prélevé par l'intermédiaire d'un soutirage latéral au niveau de la partie d'évacuation de la colonne de distillation.

6. Procédé selon la revendication 4 ou 5, le flux hydrocarboné à purifier, dont les constituants à bas point d'ébullition et les constituants à point d'ébullition élevé ont été éliminés partiellement, étant introduits dans la partie d'admission de la colonne à paroi de séparation.

7. Procédé selon l'une des revendications précédentes, un flux, qui contient les constituants à bas point d'ébullition, étant prélevé dans la colonne à paroi de séparation par l'intermédiaire de la tête de la colonne, et un flux, qui contient les constituants à point d'ébullition élevé, étant prélevé par l'intermédiaire du fond de la colonne.

8. Procédé selon la revendication 7, le flux prélevé en tête, qui contient les constituants à bas point d'ébullition, étant guidé vers un condenseur et condensé au moins partiellement dans celui-ci.

9. Procédé selon la revendication 8, la partie non condensée du flux étant déchargée du procédé.

10. Procédé selon la revendication 8 ou 9, la partie condensée du flux étant guidée en retour vers la colonne à paroi de séparation.

11. Procédé selon la revendication 10, la partie condensée guidée en retour du flux étant alimentée sur le premier plateau de séparation de la section supérieure de la colonne de distillation.

12. Procédé selon l'une des revendications précédentes, le flux hydrocarboné à purifier passant à travers un échangeur de chaleur et étant préchauffé avant l'introduction du flux dans la colonne à paroi de séparation.

13. Procédé selon la revendication 12, le chauffage dans l'échangeur de chaleur ayant lieu par échange de chaleur avec l'intermédiaire.

14. Procédé selon l'une des revendications précédentes, un agent de sorption, qui comprend un matériau support poreux, en particulier du SiO₂, qui est couvert de nickel et d'oxyde de zinc sur sa surface, étant utilisé lors de la séparation dans l'étape b).

15. Procédé selon la revendication 14, l'agent de sorption présentant la composition suivante, se complétant à 100% en poids :
▪ nickel : 15 à 65% en poids ;
▪ oxyde de zinc : 5 à 40% en poids ;
▪ dioxyde de silicium : 5 à 75% en poids ;
▪ graphite : 0 à 5% en poids ;
▪ autres composants 0 à 1% en poids.
